(19) Europäisches Patentamt
European Patent Office
Office européen des brevets

(11) Veröffentlichungsnummer: **0 043 125**
**B1**

(12) # EUROPÄISCHE PATENTSCHRIFT

(45) Veröffentlichungstag der Patentschrift: **28.11.84**

(51) Int. Cl.³: **C 07 F 5/06**, C 07 F 9/58, A 61 K 7/00

(21) Anmeldenummer: **81104984.0**

(22) Anmeldetag: **26.06.81**

(54) **Aluminiumorganische Verbindungen, Herstellung dieser Verbindungen, kosmetische Mittel, die diese Verbindungen als Wirkstoffe enthalten, sowie Verwendung dieser Mittel zur Bekämpfung oder Verhütung von Schuppen der Kopfhaut.**

(30) Priorität: **02.07.80 CH 5112/80**

(43) Veröffentlichungstag der Anmeldung: **06.01.82 Patentblatt 82/01**

(45) Bekanntmachung des Hinweises auf die Patenterteilung: **28.11.84 Patentblatt 84/48**

(84) Benannte Vertragsstaaten: **BE CH DE FR GB IT LI NL**

(56) Entgegenhaltungen:
**FR-A- 2 183 647**
**FR-A- 2 414 502**

(73) Patentinhaber: **Richardson-Vicks, Inc.**
**Ten Westport Road**
**Wilton, Conn. 06897 (US)**

(72) Erfinder: **Pauling, Horst, Dr.**
**Ruchholzstrasse 39**
**CH-4103 Bottmingen (CH)**

(74) Vertreter: **Lederer, Franz, Dr. et al**
**Patentanwälte Dr. Franz Lederer Dipl.-Ing. Reiner F. Meyer-Roxlau Lucile-Grahn-Strasse 22**
**D-8000 München 80 (DE)**

**EP 0 043 125 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft aluminiumorganische Verbindungen der allgemeinen Formel

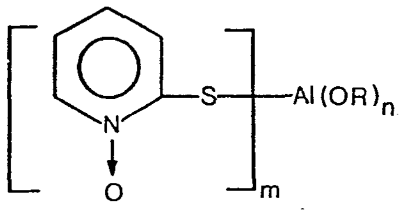

(I)

worin

m die Zahl 1 oder 2,
n die Zahl 1 oder 2,
wobei die Summe m + n 3 ist, und
R geradkettiges $C_{1-22}$-Alkyl ; mit Phenoxy monosubstituiertes geradkettiges oder verzweigtes $C_{1-4}$-Alkyl ; 2,4-Hexadienoyl ; unsubstituiertes oder mit 1 bis 5 Chloratomen oder einem Phenylrest substituiertes Phenyl ; oder eine Gruppe der Formel

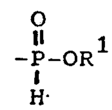

(a)

worin $R^1$ $C_{1-6}$-Alkyl, bedeuten, oder, im Falle, dass m die Zahl 1 und n die Zahl 2 bedeuten, die zwei Reste R zusammen auch die 2,2'-Methylen-bis(3,4,6-trichlor-o-phenylen)-gruppe, d. h. die Gruppe der Formel

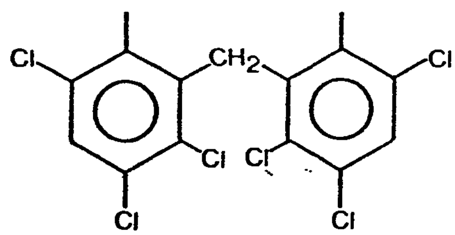

(b)

bilden, oder, im Falle, dass m die Zahl 2 und n die Zahl 1 bedeuten, R zusätzlich die 2,4,5-Trichlor-6-{2,3,5-trichlor-6-[di(2-pyridinthiolato)-aluminium]oxy-benzyl}phenyl-N,N'-dioxid-gruppe, d. h. die Gruppe der Formel

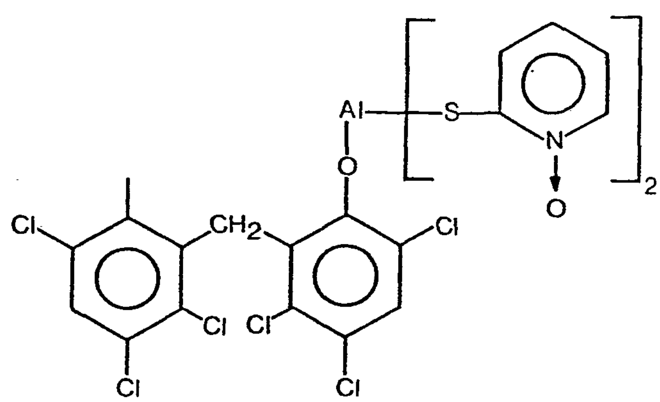

(c)

bedeutet, mit der Maßgabe, daß, wenn m = 2 ist, R nicht geradkettiges $C_{1-22}$-Alkyl bedeutet.

Die Verbindungen der Formel I besitzen antimikrobielle, insbesondere bakterizide und fungizide, Eigenschaften. Sie eignen sich insbesondere als Wirkstoffe für Haarpflegemittel, z. B. zur Bekämpfung oder Verhütung von Schuppen der Kopfhaut, bzw. für Fusspflegemittel

Die vorliegenden Verbindungen können aufgrund ihrer fungiziden Wirkung aber auch in der Landwirtschaft und im Gartenbau Verwendung finden.

Die Erfindung betrifft ferner ein Verfahren zur Herstellung von Verbindungen der Formel I, Verbindungen der Formel I als bakterizide und fungizide Mittel, kosmetische Mittel, die eine antimikrobiell wirksame Menge von mindestens einer Verbindung der Formel I als Wirkstoff enthalten sowie die Verwendung der Verbindungen bzw. Mittel als Haarpflege- bzw. Fusspflegemittel. Aus der FR-A-2 183 647, bzw. der korrespondierenden DE-A-2 248 240 ist das Bis-(N-oxypyridyl-2-thio)-isopropoxy-aluminium bekannt, welches antimikrobiell wirksam ist und welches in kosmetischen Zubereitungen und als Antischuppenmittel verwendet werden kann. Die bekannte Substanz unterscheidet sich strukturell von den erfindungsgemäßen Verbindungen.

In der Formel I können die Reste R gleich oder verschieden sein.

Beispiele von R als geradkettiges $C_{1-22}$-Alkyl sind Methyl, Aethyl, n-Propyl, n-Butyl, n-Pentyl, n-Hexyl, n-Octyl, n-Decyl, n-Hexadecyl, n-Octadecyl und n-Docosyl.

Bedeutet R geradkettiges $C_{1-22}$-Alkyl, so ist dies vorzugsweise Aethyl oder n-Hexadecyl.

Hat R die Bedeutung mit Phenoxy monosubstituiertes geradkettiges oder verzweigtes $C_{1-4}$-Alkyl, so ist dies vorzugsweise 2-Phenoxyäthyl.

R in der Bedeutung 2,4-Hexadienoyl ist vorzugsweise trans, trans-2,4-Hexadienoyl, d. h. Sorbato.

Bedeutet R mit 1 bis 5 Chloratomen substituiertes Phenyl, so ist dies vorzugsweise 2,4,6-Trichlor- oder Pentachlorphenyl. Bedeutet R mit einem Phenylrest substituiertes Phenyl, so ist dies vorzugsweise o-Phenylphenyl.

$R^1$ in der Gruppe der Formel (a) kann geradkettiges oder verzweigtes Alkyl sein, jedoch ist geradkettiges Alkyl bevorzugt. Vorzugsweise bedeutet $R^1$ Aethyl.

Im Vordergrund des Interesses stehen Verbindungen der Formel I, worin m die Zahl 2, n die Zahl 1, und R mit Phenoxy monosubstituiertes geradkettiges oder verzweigtes $C_{1-4}$-Alkyl, 2,4-Hexadienoyl, gegebenenfalls wie oben ausgeführt substituiertes Phenyl, eine Gruppe der Formel (a) oder die 2,4,5-Trichlor-6-{2,3,5-trichlor-6-[di(2-pyridinthiolato)aluminium]oxy-benzyl}phenyl-N,N'-dioxid-gruppe.

Besonders bevorzugte Verbindungen sind :

(2-Phenoxyäthoxy)bis(2-pyridinthiolato)aluminium-N,N'-dioxid,
Bis(äthylphosphonato)(2-pyridinthiolato)aluminium-N-oxid,
(Sorbato)bis(2-pyridinthiolato)aluminium-N,N'-dioxid,
(2-Biphenylyloxy)bis(2-pyridinthiolato)aluminium-N,N'-dioxid,
(Aethylphosphonato)bis(2-pyridinthiolato)aluminium-N,N'-dioxid und
[Methylen-bis(3,4,6-trichlor-o-phenylenoxy)]bis[(di-2-pyridinthiolato)]aluminium-N,N',N'',N'''-tetroxid.

In den Verbindungen der Formel I sind in gewissen Fällen asymmetrische Kohlenstoffatome vorhanden, so dass solche Verbindungen als optische Antipoden vorliegen können. Durch das Vorliegen der Doppelbindungen in den Verbindungen der Formel I, worin R 2,4-Hexadienoyl bedeutet, tritt für diese Verbindungen zusätzlich geometrische Isomerie auf. Die Formel I soll demnach all diese möglichen isomeren Formen umfassen.

Das erfindungsgemässe Verfahren zur Herstellung der Verbindungen der Formel I ist dadurch gekennzeichnet, dass man

a) eine Verbindung der Formel

$$(R^2)_3Al \qquad (II)$$

worin $R^2$ einen gesättigten, geradkettigen oder verzweigten $C_{2-6}$-Kohlenwasserstoffrest bedeutet, mit einer Verbindung der Formel

$$ROH \qquad (III)$$

worin R die zuvor angegebene Bedeutung besitzt, und mit Pyridin-N-oxid-2-thiol umsetzt, oder

b) eine Verbindung der Formel

$$(R^2)_mAlCl_n \qquad (IV)$$

worin $R^2$, m und n die zuvor angegebenen Bedeutungen besitzen, mit einer Verbindung der Formel

$$RO^{\ominus}Y^{\oplus} \qquad (V)$$

worin $Y^{\oplus}$ ein Alkalimetall-, z. B. Natrium- oder Kalium-, oder ein Ammoniumion bedeutet, und R die zuvor angegebene Bedeutung besitzt, und mit Pyridin-N-oxid-2-thiol umsetzt.

Gemäss Verfahrensvariante a) kann die Reaktion einstufig oder zweistufig durchgeführt werden. Die nachstehenden Reaktionsgleichungen stellen die zwei Möglichkeiten a) (i) und a) (ii) dar :

a) (i) $(R^2)_3Al + n' \; ROH \longrightarrow (R^2)_mAl(OR)_n + n \; R^2H$

(II) (III) (VI)

$\left((R^2)_{m+n}Al\right)$

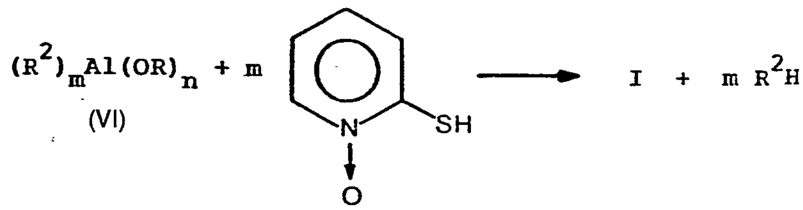

(VI)

a) (ii)

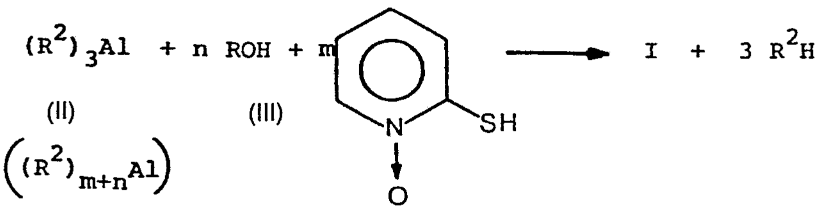

(II) (III)

$\left((R^2)_{m+n}Al\right)$

Bei der Verfahrensvariante a) (i) (erste Stufe) oder (ii) kann die Reaktion zweckmässigerweise durchgeführt werden, indem man die betreffenden Ausgangsmaterialien der Formeln II und III, und im Falle der Verfahrensvariante a) (ii) auch das Ausgangsmaterial Pyridin-N-oxid-2-thiol, in einem inerten Lösungsmittel reagieren lässt. Beispiele geeigneter Lösungsmittel sind geradkettige oder verzweigte gesättigte aliphatische Kohlenwasserstoffe wie n-Hexan und Isooctan, gegebenenfalls substituierte aromatische Kohlenwasserstoffe wie Benzol und Toluol, halogenierte Kohlenwasserstoffe wie Methylenchlorid und Chloroform, und Aether oder ätherartige Verbindungen wie Diäthyläther, Dioxan und Anisol. Da die Ausgangsmaterialien der Formeln II und III und die Zwischenprodukte der Formel VI sehr hydrolyseempfindlich sind und diese Verbindungen sowie Pyridin-N-oxid-2-thiol darüber hinaus oxydationsempfindlich sind, muss die Reaktion unter möglich wasser- und luftfreien Bedingungen durchgeführt werden. Es werden daher wasserfreie Lösungsmittel verwendet, und die Reaktionen werden zweckmässigerweise in trockener Schutzgasatmosphäre, z. B. Stickstoff oder Argon, durchgeführt.

Die Reaktionstemperaturen liegen zweckmässigerweise in einem Bereich zwischen −20 °C und 100 °C, vorzugsweise zwischen 0 °C und 60 °C.

Bei der Verfahrensvariante a) (i) können beide Stufen unter analogen Reaktionsbedingungen, wie oben erwähnt, durchgeführt werden. Eine Isolierung des Produktes der ersten Stufe, d. h. der Verbindung der Formel VI, ist daher nicht notwendig, und dasselbe Lösungsmittel, eventuell unter Zusatz einer weiteren Menge davon, kann in der zweiten Stufe benützt werden wie in der ersten. Nach Einsatz des Pyridin-N-oxid-2-thiols wird die Reaktion der zweiten Stufe in Gang gebracht.

Die Mengen der in den Verfahrensvariante a) zu verwendenden Ausgangsmaterialien sind zweckmässigerweise stöchiometrisch, und die Ausgangsmaterialien selbst werden vorzugsweise in einem möglichst reinen Zustand verwendet. Unter solchen Umständen, und da die Reaktionen in der Regel völlig quantitativ verlaufen und das einzige Nebenprodukt der leicht flüchtige niedrige Kohlenwasserstoff $R^2H$ ist, fällt das Endprodukt normalerweise in hoher Reinheit an und bedarf nach Entfernung des Lösungsmittels, z. B. durch Abdampfen, normalerweise keiner zusätzlichen Reinigung mehr.

Bei der Verfahrensvariante b) wird die Reaktion vorzugsweise in zwei Stufen durchgeführt. Die nachstehenden Reaktionsgleichungen stellen das zweistufige Verfahren dar:

b) $(R^2)_mAlCl_n + n \; RO^{\ominus} Y^{\oplus} \longrightarrow (R^2)_mAl(OR)_n + nY^{\oplus} Cl^{\ominus}$

(IV) (V) (VI)

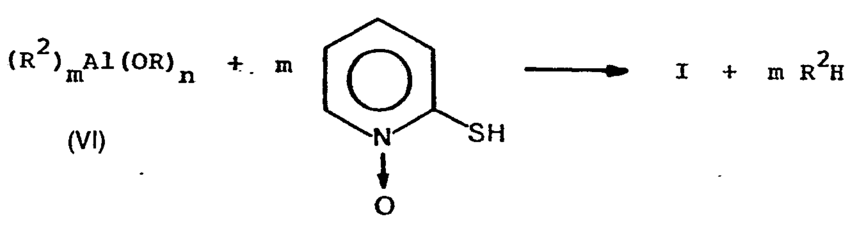

(VI)

Die geeigneten Reaktionsbedingungen, z. B. Lösungsmittel, Ausschluss von Wasser, Schutzgasatmosphäre, Reaktionstemperaturen und Ausgangsmaterialienverhältnisse, sind im allgemeinen die bezüglich der Verfahrensvariante a) oben erwähnten. Bei der Verfahrensvariante b) wird zweckmässigerweise der angefallene Niederschlag des Salzes der Formel $Y^{\oplus}Cl^{\ominus}$ nach Beendigung der ersten Reaktionsstufe abgetrennt, z. B. durch Filtrierung des Reaktionsgemisches. Das weitere Vorgehen kann wie für die Verfahrensvariante a) beschrieben erfolgen. Auch in diesem Fall verlaufen die Reaktionen in der Regel völlig quantitativ, so dass eine Reinigung des Endproduktes normalerweise unnötig ist.

Das in den obigen Verfahrensvarianten a) und b) verwendete Pyridin-N-oxid-2-thiol ist nicht unbeschränkt haltbar. In der Luft tritt z. B. langsame Oxydation zum entsprechenden Disulfid ein. Aus diesem Grunde wird vorteilhaft das freie Thiol vor jeder Umsetzung frisch aus dem stabilen und käuflichen Natriumthiolat durch Ansäuern gewonnen.

Die als Ausgangsmaterialien verwendbaren Verbindungen der Formeln II, III, IV und V sind entweder bekannt oder können nach an sich bekannten Methoden hergestellt werden.

Wie es bei vielen bekannten Aluminiumverbindungen der Fall ist, kann das Aluminiumatom in den Verbindungen der Formel I unter Zuhilfenahme von sogenannten Nebenvalenzbindungen Raumstrukturen ausbilden, bei denen das Aluminiumatom höhere Koordinationszahlen, z. B. 4 oder 6, aufweist. Die entstehenden komplexen Strukturen weisen in der Regel kein einheitliches Molgewicht auf, sondern sind Oligomere bzw. Polymere der jeweiligen monomeren Verbindungen. Aus diesem Grunde sind die Schmelzpunkte der betreffenden Verbindungen im allgemeinen nicht charakteristisch ; in den meisten Fällen besitzen die Verbindungen hingegen einen relativ eng begrenzten Schmelbereich, in dem oftmals Zersetzung des betreffenden Produktes stattfindet.

Die Verbindungen der Formel I besitzen antimikrobielle Wirkung. Ein Beispiel von Pilzstämmen, gegen welche die Verbindungen der Formel I wirksam sind, ist Pityrosporum ovale. Dieser Stamm ist hauptsächlich für die Schuppenbildung auf der Kopfhaut verantwortlich.

Unter den Bakterienstämmen, gegen welche die Verbindungen der Formel I wirksam sind, sollen insbesondere Staphylococcus aureus, Escherichia coli, Pseudomonas aeruginosa und Mycobakterium tuberculosis genannt werden.

Die Verbindungen der Formel I sind wegen ihrer bakteriziden und fungiziden Aktivität besonders geeignet als Wirkstoffe für kosmetische Mittel, und zwar für Haarpflege- und Fusspflegemittel. Zu diesem Zweck können die Verbindungen der Formel I zu verschiedenartigen Mitteln formuliert werden. Das erfindungsgemässe kosmetische Mittel ist dadurch gekennzeichnet, dass es eine antimikrobiell wirksame Menge von mindestens einer Verbindung der allgemeinen Formel I, wie oben definiert, sowie in Haarpflege- bzw. Fusspflegemitteln gebräuchliches Trägermaterial bzw. anderweitig kosmetisch wirksames Begleitmittel enthält. Solche Mteel sind z. B. Shampoos, Haarwässer, Frisiermittel (Frisiergels), Sprays und Lotionen. Die Mittel liegen vorzugsweise in flüssiger Form vor.

In den erfindungsgemässen Mitteln betragen die Konzentrationen an Wirkstoff der Formel I im allgemeinen 0,001 bis 5 Gewichtsprozent, vorzugsweise 0,01 bis 3 Gewichtsprozent.

Liegen die Mittel in Form von Shampoos vor, so beträgt die Konzentration an Wirkstoff der Formel I vorzugsweise 0,05 bis 2, insbesondere 0,1 bis 1, Gewichtsprozent. Die bevorzugten Konzentrationen in Haarwässern und Frisiermitteln liegen bei etwa 0,005 bis 0,2, insbesondere bei etwa 0,1 bis 0,1 Gewichtsprozent. Im Falle von Lotionen, die in Form von wässrigen Lösungen, jedoch bevorzugt wässrigalkoholischen Lösungen, vorliegen, beträgt die Konzentration an Wirkstoff der Formel I vorzugsweise 0,01 bis 0,1 Gewichtsprozent. Sprays, die normalerweise als alkoholische Lösung mit Treibgas formuliert sind, enthalten vorzugsweise 0,01 bis 3, insbesondere 0,05 bis 2, Gewichtsprozent an Wirkstoff der Formel I, bezogen auf das Gesamtgewicht des Aerosolgemisches.

Es versteht sich von selbst, dass die einzelnen Ausführungsformen der erfindungsgemässen kosmetischen Mittel, wie Shampoos, Lotionen oder Sprays, allgemein übliche Bestandteile derartiger kosmetischer Formulierungen, z. B. Waschmittel, Schutzmittel, Antioxydantien, Parfüme, enthalten können. In den Lotionen und Sprays können z. B. als Alkohole insbesondere Aethanol oder Isopropanol benützt werden, wobei die wässrigalkoholischen Lösungen vorzugsweise 30 bis 80 Gewichtsprozent Alkohol enthalten. Für Sprays sind alle bei Aerosolgemischen herkömmlichen Treibgase, z. B. halogenierte Kohlenwasserstoffe, verwendbar.

Das erfindungsgemässe Verfahren zur Bekämpfung oder Verhütung von Schuppen der Kopfhaut ist dadurch gekennzeichnet, dass man die Kopfhaut mit einer wirksamen Menge des erfindungsgemässen Haarpflegemittels behandelt.

Die folgenden Beispiele dienen zur näheren Erläuterung der Erfindung.

I. Herstellung der Wirkstoffe :

### Beispiel 1

(2-Biphenylyloxy)bis(2-pyridinthiolato)aluminium-N,N'-dioxid

16 g Triisobutylaluminium und 13,72 g 2-Phenyl-phenol werden in absolutem Toluol zum (2-Biphenylyloxy)diisobutylaluminium und dieses dann mit 20,52 g Pyridin-N-oxid-2-thiol zur Titelverbindung umgesetzt. Die Ausbeute des Produktes, eines fast weissen Pulvers, beträgt 33,4 g (ca. 92,5 % der Theorie). Beim Erhitzen sintert es ab ca. 174 °C und schmilzt bei 236-238 °C.
Mikroanalyse :

| | | | | |
|---|---|---|---|---|
| berechnet : | % C 58,92 | % H 3,82 | % N 6,25 | % S 14,30 |
| gefunden : | % C 58,98 | % H 3,91 | % N 6,22 | % S 14,34 |

Beispiel 2

(2,4,6-Trichlorphenoxy)bis(2-pyridinthiolato)aluminium-N,N'-dioxid

17,5 g Triisobutylaluminium und 17,4 g 2,4,6-Trichlorphenol werden in absolutem Toluol zum (2,4,6-Trichlorphenoxy)diisobutylaluminium und dieses dann mit 22,4 g Pyridin-N-oxid-2-thiol zur Titelverbindung umgesetzt. Die Ausbeute des Produktes, eines feinen weissen Pulvers mit einem Schmelzpunkt von 207-208 °C, beträgt 28,6 g (ca. 68 % der Theorie).
Mikroanalyse :

| | | | | |
|---|---|---|---|---|
| berechnet : | % C 40,4 | % H 2,12 | % S 13,48 | % N 5,89 |
| gefunden : | % C 41,0 | % H 2,21 | % S 13,56 | % N 5,60 |

Beispiel 3

[Methylen-bis(3,4,6-trichlor-o-phenylenoxy)](2-pyridinthiolato)aluminium-N-oxid

Zu einer Lösung von 8,96 g frisch hergestelltem Pyridin-N-oxid-2-thiol und 28,5 g Hexachlorophen in 250 g absolutem Diäthyläther bei − 10 °C wird eine Lösung von 13,9 g Triisobutylaluminium in 30 ml absolutem n-Hexan tropfenweise gegeben, wobei sich ein weisser Niederschlag bildet. Das Reaktionsgemisch wird 16 Stunden bei Raumtemperatur und danach 1 Stunde bei Rückflusstemperatur gerührt. Anschliessend wird der Niederschlag abfiltriert, zweimal mit einer Mischung von Diäthyläther und n-Hexan ausgewaschen und 4 Stunden im Hochvakuum getrocknet. Man erhält 35,7 g (ca. 91,5 % der theoretischen Ausbeute) eines weissen Pulvers, das bei 228-230 °C unter Zersetzung schmilzt.
Mikroanalyse :

| | | | | |
|---|---|---|---|---|
| berechnet : | % C 38,74 | % H 1,45 | % N 2,51 | % S 5,74 |
| gefunden : | % C 38,99 | % H 1,45 | % N 2,58 | % S 6,08 |

Beispiel 4

[Methylen-bis-(3,4,6-trichlor-o-phenylenoxy)]bis-[(di-2-pyridinthiolato)]aluminium-N,N',N'',N'''-tetroxid

Analog dem im Beispiel 3 beschriebenen Verfahren werden 32,46 g Pyridin-N-oxid-2-thiol, 25,9 g Hexachlorophen und 25,2 g Triisobutylaluminium zur Titelverbindung umgesetzt. Die Isolierung des Produktes erfolgt in analoger Weise. Man erhält 56,9 g (ca. 92,5 % der theoretischen Ausbeute) eines feinen weissen Pulvers mit einem Schmelzintervall von 208-210 °C.
Mikroanalyse :

| | | | | |
|---|---|---|---|---|
| berechnet : | % C 41,14 | % H 2,09 | % N 5,82 | % S 13,31 |
| gefunden : | % C 41,67 | % H 2,16 | % N 6,00 | % S 13,42 |

Beispiel 5

Bis(2-phenoxyäthoxy)(2-pyridinthiolato)aluminium-N-oxid

Analog dem im Beispiel 3 beschriebenen Verfahren werden 16,23 g Pyridin-N-oxid-2-thiol, 35,2 g 2-Phenoxyäthanol und 14,5 g Triäthylaluminium zur Titelverbindung umgesetzt. Die Isolierung des Produktes erfolgt in analoger Weise. Man erhält 50,9 g (ca. 93 % der theoretischen Ausbeute) Kristalle mit einem Schmelzpunkt von 52-55 °C.
Mikroanalyse :

| | | | | |
|---|---|---|---|---|
| berechnet : | % C 59,01 | % H 5,19 | % N 3,28 | % S 7,50 |
| gefunden : | % C 59,40 | % H 5,25 | % N 3,07 | % S 7,51 |

Beispiel 6

(2-Phenoxyäthoxy)bis(2-pyridinthiolato)aluminium-N,N'-dioxid

Analog dem im Beispiel 3 beschriebenen Verfahren werden 20,5 g Pyridin-N-oxid-2-thiol, 11,15 g 2-Phenoxyäthanol und 22,8 g Trihexylaluminium zur Titelverbindung umgesetzt. Die Isolierung erfolgt in analoger Weise. Man erhält 31,3 g (ca. 93,1 % der theoretischen Ausbeute) eines lockeren weisslichen Pulvers mit einem Schmelzintervall von 249-252 °C (unter Zersetzung).
Mikroanalyse :

| | | | | |
|---|---|---|---|---|
| berechnet : | % C 51,92 | % H 4,11 | % N 6,73 | % S 15,40 |
| gefunden : | % C 51,90 | % H 4,10 | % N 6,80 | % S 15,69 |

Beispiel 7

Diphenoxy(2-pyridinthiolato)aluminium-N-oxid

17,5 g Triisobutylaluminium und 16,6 g Phenol werden zum Diphenoxyaluminiumisopropoxylat und dieses dann mit 11,2 g Pyridin-N-oxid-2-thiol zur Titelverbindung umgesetzt. Man erhält 22,5 g (ca. 75,4 % der theoretischen Ausbeute) eines feinen weissen Pulvers mit einem Schmelzpunkt von 233-234,5 °C.
Mikroanalyse :

| | | | | |
|---|---|---|---|---|
| berechnet : | % C 60,17 | % H 4,16 | % N 4,13 | % S 9,45 |
| gefunden : | % C 60,53 | % H 4,28 | % N 4,10 | % S 9,52 |

Beispiel 8

Bis(2-biphenylyloxy)(2-pyridinthiolato)aluminium-N-oxid

17,5 g Triisobutylaluminium und 30 g 2-Phenyl-phenol werden zum Bis(2-biphenylyloxy)isobutylaluminium und dieses dann mit 11,2 g Pyridin-N-oxid-2-thiol zur Titelverbindung umgesetzt. Es werden 30,25 g (ca. 69,9 % der theoretischen Ausbeute) eines weissen feinkörnigen Pulvers mit einem Schmelzpunkt von 219-221 °C erhalten.
Analyse :

| | | | | |
|---|---|---|---|---|
| berechnet : | % C 70,86 | % H 4,51 | % N 2,85 | % S 6,52 |
| gefunden : | % C 71,86 | % H 4,35 | % N 3,00 | % S 6,45 |

Beispiel 9

Bis(n-butylphosphonato)(2-pyridinthiolato)aluminium-N-oxid

12,9 g Triisobutylaluminium und 18,0 g Mono-n-butylester der phosphorigen Säure werden zum Bis(-n-butylphosphonato)isobutylaluminium und dieses dann mit 8,26 g Pyridin-N-oxid-2-thiol zur Titelverbindung umgesetzt. Es werden 23,2 g (ca. 83,6 % der theoretischen Ausbeute) eines weisslichen Pulvers mit einem Schmelzpunkt von 227-232 °C (unter Zersetzung) erhalten.
Mikroanalyse :

| | | | | | |
|---|---|---|---|---|---|
| berechnet : | % C 36,54 | % H 5,66 | % N 3,28 | % S 7,50 | % P 14,50 |
| gefunden : | % C 35,51 | % H 5,32 | % N 3,43 | % S 7,79 | % P 14,19 |

Beispiel 10

(n-Butylphosphonato)bis(2-pyridinthiolato)aluminium-N,N'-dioxid

12,9 g Triisobutylaluminium und 8,98 g Mono-n-butylester der phosphorigen Säure werden zum Bis(isobutyl)(n-butylphosphonato)aluminium und dieses dann mit 16,5 g Pyridin-N-oxid-2-thiol zur Titelverbindung umgesetzt. Man erhält 22,8 g (ca. 84,2 % der theoretischen Ausbeute) gelbliche Kristalle mit einem Schmelzintervall von 224-228 °C (unter Zersetzung).
Mikroanalyse :

| | | | | | |
|---|---|---|---|---|---|
| berechnet : | % C 40,38 | % H 4,36 | % N 6,73 | % S 15,40 | % P 7,44 |
| gefunden : | % C 39,21 | % H 4,16 | % N 6,86 | % S 15,84 | % P 6,86 |

Beispiel 11

Bis(äthylphosphonato)(2-pyridinthiolato)aluminium-N-oxid

10 g 98 %igem Diäthylphosphit bei 0 °C werden 50 ml 25 %iges wässriges Ammoniak unter Rühren

zugetropft. Man lässt 1 Stunde bei Raumtemperatur weiterrühren und destilliert die flüchtigen Bestandteile (Ammoniak, Aethanol und Wasser) bei 50 °C Badtemperatur und unter vermindertem Druck ab. Zum Rückstand werden zur azeotropen Entfernung des noch anhaftenden Wassers zweimal je 100 ml absolutes Toluol gegeben und dieses bei 50 °C im Vakuum jeweils wieder abdestilliert. Der Inhalt des Reaktionskolbens wird 24 Stunden bei 50 °C im Hochvakuum getrocknet. Das Produkt besteht aus dem Ammoniumsalz des Monoäthylesters der phosphorigen Säure.

Zum obigen Kolbeninhalt werden 200 ml absolutes Toluol gegeben, und die entstandene Suspension wird eine halbe Stunde bei 50 °C gerührt. Danach lässt man das Reaktionsgemisch auf Raumtemperatur abkühlen und tropft eine Lösung von 5,5 g Isobutylaluminiumdichlorid in 70 ml absolutem Toluol während 1 Stunde dazu. Man erwärmt das Reaktionsgemisch 1 Stunde auf 60 °C, lässt es auf 20 °C abkühlen und filtriert das ausgefallene Ammoniumchlorid unter Luft- und Feuchtigkeitsausschluss ab. Das Ammoniumchlorid wird zweimal mit je 10 ml absolutem Toluol ausgewaschen. Zum vereinigten Filtrat wird eine Lösung von 4,5 g Pyridin-N-oxid-2-thiol in 50 ml absolutem Toluol innert 1 Stunde bei Raumtemperatur zugetropft. Man rührt das Gemisch 1 Stunde bei 40 °C und 60 Stunden bei Raumtemperatur. Dabei fällt das gewünschte Endprodukt aus. Das Toluol wird bei 50 °C unter vermindertem Druck abdestilliert und das Produkt zweimal mit je 25 ml absolutem n-Hexan ausgewaschen und anschliessend 12 Stunden im Hochvakuum bei 50 °C getrocknet. Man erhält 11,3 g (ca. 86 % der theoretischen Ausbeute) der Titelverbindung als gelbliches Kristallisat, das bei 208-210 °C unter Zersetzung schmilzt.

Mikroanalyse :

| | | | | |
|---|---|---|---|---|
| berechnet : | % C 29,12 | % H 4,34 | % N 3,77 | % S 8,64 |
| gefunden : | % C 29,76 | % H 4,48 | % N 3,97 | % S 8,86 |

Beispiel 12

(Aethylphosphonato)bis(2-pyridinthiolato)aluminium-N,N'-dioxid

Analog dem im ersten Abschnitt des Beispiels 11 beschriebenen Verfahren wird aus Diäthylphosphit und Natronlauge das Natriumsalz des Monoäthylesters der phosphorigen Säure hergestellt. 2,72 g dieses Salzes werden analog dem im zweiten Abschnitt des Beispiels 11 beschriebenen Verfahren mit 3,64 g Diisobutylaluminiumchlorid und 5,24 g Pyridin-N-oxid-2-thiol umgesetzt. Auf diese Weise erhält man 7,0 g (ca. 87,5 % der theoretischen Ausbeute) der Titelverbindung als gelbbräunliches Kristallisat mit einem Schmelzintervall von 208-220 °C (unter Zersetzung).

Mikroanalyse :

| | | | | | |
|---|---|---|---|---|---|
| berechnet : | % C 37,12 | % H 3,63 | % N 7,21 | % S 16,51 | % P 7,98 |
| gefunden : | % C 37,77 | % H 3,90 | % N 6,87 | % S 16,81 | % P 8,45 |

Beispiel 13

(Sorbato)bis(2-pyridinthiolato)aluminium-N,N'-dioxid

12,2 g Triisobutylaluminium und 6,9 g Sorbinsäure werden in absolutem Toluol zu Sorbato-diisobutylaluminium und dieses dann mit 15,6 g Pyridin-N-oxid-2-thiol zur Titelverbindung umgesetzt. Das weisse Produkt fällt dabei in einer Ausbeute von 22,7 g (ca. 93 % der theoretischen Ausbeute) an und schmilzt beim Erhitzen ab ca. 270 °C unter Zersetzung.

Mikroanalyse :

| | | | | |
|---|---|---|---|---|
| berechnet : | % C 49,23 | % H 3,87 | % N 7,18 | % S 16,42 |
| gefunden : | % C 49,45 | % H 3,80 | % N 6,99 | % S 16,46 |

Beispiel 14

Phenoxy-bis(2-pyridinthiolato)aluminium-N,N'-dioxid

Triisobutylaluminium und Phenol werden in absolutem Toluol zum Phenoxy-diisobutylaluminium und dieses dann mit Pyridin-N-oxid-2-thiol zur Titelverbindung umgesetzt. Die Ausbeute des Produktes, eines weissen Feststoffes, beträgt 98 % der Theorie. Schmelzpunkt : 240-241 °C (unter Zersetzung).

Mikroanalyse :

| | | | | |
|---|---|---|---|---|
| berechnet : | % C 51,61 | % H 3,52 | % N 7,52 | % S 17,22 |
| gefunden : | % C 51,79 | % H 3,72 | % N 7,36 | % S 17,02 |

Beispiel 15

Pentachlorphenoxy-bis(2-pyridinthiolato)aluminium-N,N'-dioxid

Triisobutylaluminium und Pentachlorphenol werden in absolutem Toluol zum Pentachlorphenoxydiisobutylaluminium und dieses dann mit Pyridin-N-oxid-2-thiol zur Titelverbindung umgesetzt. Die Ausbeute des Produktes, eines weissen Feststoffes, beträgt 89 % der Theorie. Schmelzpunkt : 231-232 °C (unter Zersetzung).

Mikroanalyse :

| | | | | | |
|---|---|---|---|---|---|
| berechnet : | % C 35,29 | % H 1,48 | % N 5,14 | % Cl 32,55 | % S 11,77 |
| gefunden : | % C 35,43 | % H 1,67 | % N 5,18 | % Cl 32,19 | % S 12,01 |

II. Herstellung des Ausgangsmaterials Pyridin-N-oxid-2-thiol :

Beispiel 16

Zu einer Lösung von 25 g Pyridin-N-oxid-2-thiol-Natriumsalz (z. B. Natrium-Omadin® der Firma Olin Corp., Rochester, N.Y., USA) in 130 ml entionisiertem Wasser wird langsam und unter gutem Rühren eine Lösung von 9,65 ml Eisessig in 25 ml entionisiertem Wasser gegeben. Dabei fällt das Pyridin-N-oxid-2-thiol aus. Dieses Produkt wird abfiltriert und danach zweimal mit je 50 ml eiskaltem entionisiertem Wasser gewaschen.

Zum Filtrat werden 2 ml Eisessig gegeben, und die wässrige Lösung wird anschliessend zweimal mit je 100 ml Diäthyläther extrahiert. Die vereinigten Aetherphasen werden unter vermindertem Druck eingeengt, und zum Rückstand wird das abfiltrierte feuchte Produkt gegeben. Das dem Produkt anhaftende Wasser wird danach mit absolutem Toluol azeotropisch entfernt, indem man zweimal mit je 25 ml Toluol bei ca. 50 °C unter vermindertem Druck die Lösung einengt oder man das Produkt bei ca. 35 °C unter vermindertem Druck trocknen lässt.

Es werden 16 g (ca. 74,5 % der theoretischen Ausbeute) Pyridin-N-oxid-2-thiol als hellgrau-braunes kristallines Material erhalten, das baldmöglichst zu den jeweiligen gewünschten Endprodukten der Formel I umgesetzt werden soll.

III. Formulierungsbeispiele :

Beispiel 17

Eine Antischuppenlotion hat die folgende Zusammensetzung :

| | |
|---|---|
| 1 000 ml enthalten | |
| (2-Phenoxyäthoxy)bis(2-pyridinthiolato)-aluminium-N,N'-dioxid | 0,5 g |
| Feinsprit (94 %ig) | 300,0 g |
| Cystein-hydrochlorid | 1,0 g |
| D-Panthenol | 5,0 g |
| Citronensäure, wasserfrei | 1,0 g |
| Triäthanolamin (50 gewichtsprozentig) | 7,6 g |
| Destilliertes oder entmineralisiertes Wasser | ad 1 000 ml |

Beispiel 18

Ein Fusspflegemittel (in Form einer Lotion) hat die folgende Zusammensetzung :

| | |
|---|---|
| 1 000 ml enthalten | |
| eine Paste bestehend aus Cremophor® RH 60 (Solubilisierungsmittel : | |
| hydriertes Ricinusöl/Aethylenoxid (1 : 60)-Addukt) und | 2,0 g |
| (2-Biphenylyloxy)bis(2-pyridinthiolato) aluminium-N,N'-dioxid | 0,5 g |
| Aethanol (94 Gewichtsprozent) | 400 ml |
| d,1-α-Tocopherol | 0,1 g |
| Destilliertes Wasser | ad 1 000 ml |

Die oben erwähnte Paste wird durch Schmelzen des Cremphor® RH 60, Zugabe der Aluminiumverbindung und anschliessendes Homogenisieren hergestellt. Nach Lösung der Paste im Aethanol werden weiterhin das d,1-α-Tocopherol und destilliertes Wasser zugegeben.

Beispiel 19

Ein Fusspflegemittel (in Form eines Sprays) hat die folgende Zusammensetzung :

| | |
|---|---|
| Phenoxy-bis(2-pyridinthiolato)-aluminium-N,N'-dioxid | 0,2 g |
| Methanol | 16,0 g |
| Destilliertes Wasser | 24,0 g |
| Treibgas (eine 1 : 1-Mischung von Dichlordifluormethan und Dichlortetrafluoräthan) | ad 100,0 g |

IV. Biologische Befunde :

Beispiel 20

Wirkung gegen Pityrosporum ovale (Schuppen)

Lösungen der zu prüfenden Verbindungen in verschiedenen Konzentrationen werden auf einen Nährboden gegeben. Als Lösungsmittel dient Wasser oder Polyäthylenglykol. Die Oberfläche des Nährbodens wird mit einer Suspension von Pityrosporum ovale Stamm Nr. 1 bestrichen und bei 25 °C bebrütet. Nach 5 Tagen wird das Hefenwachstum mit blossem Auge untersucht. Die Ergebnisse sind in der nachstehenden Tabelle 1 festgehalten.

Tabelle 1

| Verbindung (Beispiel Nr.) | Konzentration (µg/ml), bei der noch Wachstum festgestellt ist | Konzentration (µg/ml), bei der kein Wachstum mehr festzustellen ist |
|---|---|---|
| (2-Phenoxyäthoxy)bis(2-pyridinthiolato)aluminium-N,N'-dioxid (6) | 0,1 | 1 |
| Bis(äthylphosphonato) (2-pyridinthiolato)aluminium-N-oxid (11) | 0,1 | 1 |
| (Aethylphosphonato)bis(2-pyridinthiolato)aluminium-N,N'-dioxid (12) | 1 | 10 |
| (2-Biphenylyloxy)bis(2-pyridinthiolato)aluminium-N,N'-dioxid (1) | 1 | 10 |
| (Sorbato)bis(2-pyridinthiolato)aluminium-N,N'-dioxid (13) | 1 | 10 |
| Hexachlorophen } Stand der Technik | 10 | 100 |
| Tri(2-pyridinthiolato)-aluminium } Stand der Technik | 1 | 10 |

**Ansprüche**

1. Verbindungen der allgemeinen Formel

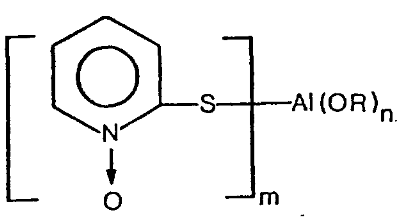

(I)

worin

m die Zahl 1 oder 2,

n die Zahl 1 oder 2,

wobei die Summe m + n 3 ist, und

R geradkettiges $C_{1-22}$-Alkyl ; mit Phenoxy monosubstituiertes geradkettiges oder verzweites $C_{1-4}$-Alkyl ; 2,4-Hexadienoyl ; unsubstituiertes oder mit 1 bis 5 Chloratomen oder einem Phenylrest substituiertes Phenyl ; oder eine Gruppe der Formel

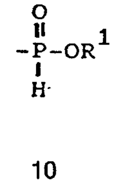

(a)

worin $R^1$ $C_{1-6}$-Alkyl, bedeuten, oder, im Falle, dass m die Zahl 1 und n die Zahl 2 bedeuten, die zwei Reste R zusammen auch die 2,2'-Methylen-bis(3,4,6-trichlor-o-phenylen)-gruppe bilden, oder, im Falle, dass m die Zahl 2 und n die Zahl 1 bedeuten, R zusätzlich die 2,4,5-Trichlor-6-{2,3,5-trichlor-6-[di(2-pyridinthiolato)aluminium]oxy-benzyl}phenyl-N,N'-dioxid-gruppe bedeutet, mit der Maßgabe, daß, wenn m = 2 ist, R nicht geradkettiges $C_{1-22}$-Alkyl bedeutet.

2. Verbindungen nach Anspruch 1, bei denen m = 2 und n = 1 ist und R mit Phenoxy monosubstituiertes geradkettiges oder verzweigtes $C_{1-4}$-Alkyl, 2,4-Hexadienoyl, gegebenenfalls mit 1,5-Chloratomen substituiertes Phenyl, eine Gruppe der in Anspruch 1 wiedergegebenen Formel (a) oder die 2,4,5-Trichlor-6-{2,3,5-trichlor-6-[di(2-pyridinthiolato)aluminium]oxybenzyl}phenyl-N,N'-dioxid-gruppe bedeutet.

3. Verbindungen nach Anspruch 1, worin m die Zahl 1 und n die Zahl 2 bedeuten.

4. (2-Phenoxyäthoxy)bis(2-pyridinthiolato)aluminium-N,N'-dioxid.

5. Bis(äthylphosphonato)(2-pyridinthiolato)aluminium-N-oxid.

6. (2-Biphenylyloxy)bis(2-pyridinthiolato)aluminium-N,N'-dioxid.

7. [Methylen-bis(3,4,6-trichlor-o-phenylenoxy]bis[(di-2-pyridinthiolato)]aluminium-N,N',N'',N'''-tetroxid.

8. Eine Verbindung nach Anspruch 1, ausgewählt unter :

(2,4,6-Trichlorphenoxy)bis(2-pyridinthiolato)aluminium-N,N'-dioxid,
[Methylen-bis(3,4,6-trichlor-o-phenylenoxy)](2-pyridinthiolato)aluminium-N-oxid,
Bis(2-phenoxyäthoxy)(2-pyridinthiolato)aluminium-N-oxid,
Diphenoxy(2-pyridinthiolato)aluminium-N-oxid,
Bis(2-biphenylyloxy)(2-pyridinthiolato)aluminium-N-oxid,
(Sorbato)bis(2-pyridinthiolato)aluminium-N,N'-dioxid,
Phenoxy-bis(2-pyridinthiolato)aluminium-N,N'-dioxid und
Pentachlorphenoxy-bis(2-pyridinthiolato)aluminium-N,N'-dioxid.

9. Eine Verbindung nach Anspruch 1, ausgewählt unter :

(n-Butylphosphonato)bis(2-pyridinthiolato) aluminium-N,N'-dioxid
(Aethylphosphonato)bis(2-pyridinthiolato)aluminium-N-oxid und
Bis(n-butylphosphonato)(2-pyridinthiolato)aluminium-N-oxid.

10. Verbindungen nach einem der Ansprüche 1 bis 9 als antimikrobielle Mittel.

11. Kosmetisches Mittel, dadurch gekennzeichnet, dass es eine antimikrobiell wirksame Menge von mindestens einer Verbindung nach einem der Ansprüche 1 bis 9 enthält.

12. Kosmetisches Mittel nach Anspruch 11, dadurch gekennzeichnet, dass es eine antimikrobiell wirksame Menge von (2-Phenoxyäthoxy)bis(2-pyridinthiolato)aluminium-N,N'-dioxid enthält.

13. Kosmetisches Mittel nach Anspruch 11, dadurch gekennzeichnet, dass es eine antimikrobielle wirksame Menge von Bis(äthylphosphonato) (2-pyridinthiolato)aluminium-N-oxid enthält.

14. Kosmetisches Mittel nach Anspruch 11, dadurch gekennzeichnet, dass es eine antimikrobiell wirksame Menge von (2-Biphenylyloxy)bis(2-pyridinthiolato)aluminium-N,N'-dioxid enthält.

15. Kosmetisches Mittel nach Anspruch 11, dadurch gekennzeichnet, dass es eine antimikrobiell wirksame Menge von [Methylen-bis(3,4,6-trichlor-o-phenylenoxy)]bis[(di-2-pyridinthiolato)]aluminium-N,N',N'',N'''-tetroxid enthält.

16. Verfahren zur Herstellung der Verbindungen nach Anspruch 1, dadurch gekennzeichnet, dass man

a) eine Verbindung der Formel

$$(R^2)_3Al \qquad (II)$$

worin $R^2$ einen gesättigten geradkettigen oder verzweigten $C_{2-6}$-Kohlenwasserstoffrest bedeutet, mit einer Verbindung der Formel

$$ROH \qquad (III)$$

worin R die angegebene Bedeutung besitzt, und mit Pyridin-N-oxid-2-thiol umsetzt, oder

b) eine Verbindung der Formel

$$(R^2)_mAlCl_n \qquad (IV)$$

worin $R^2$, m und n die angegebenen Bedeutungen besitzen, mit einer Verbindung der Formel

$$RO^{\ominus}Y^{\oplus} \qquad (V)$$

worin $Y^{\oplus}$ ein Alkalimetall- oder ein Ammoniumion bedeutet, und R die angegebene Bedeutung besitzt, und mit Pyridin-N-oxid-2-thiol umsetzt.

17. Verfahren zur Bekämpfung oder Verhütung von Schuppen der Kopfhaut, dadurch gekennzeichnet, dass man die Kopfhaut mit einer wirksamen Menge eines der in den Ansprüchen 11 bis 15 genannten kosmetischen Mittel behandelt.

18. Verwendung der Verbindungen der Ansprüche 1 bis 9 als Haarpflege- bzw. Fusspflegemittel.

## Claims

1. Compounds of the general formula

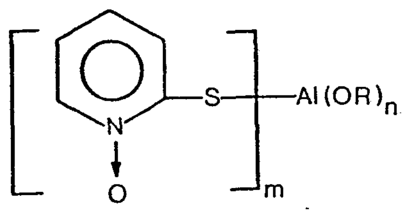

(I)

wherein
m is the number 1 or 2, and
n is the number 1 or 2,
the sum m + n being 3, and
R means straight-chain $C_{1-22}$-alkyl ; phenoxy monosubstituted straight-chain or branched $C_{1-4}$-alkyl ; 2,4-hexadienoyl ; unsubstituted phenyl or phenyl substituted with 1 to 5 chlorine atoms or a phenyl residue ; or a group having the formula

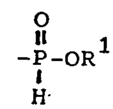

(a)

wherein $R^1$ means $C_{1-6}$-alkyl, or, in the case where m means the number 1 and n the number 2, the two residues R together also form 2,2'-methylene-bis(3,4,6-trichloro-o-phenylene) group, or, in the case where m means the number 2 and n the number 1, R also means the 2,4,5-trichloro-6-{2,3,5-trichloro-6-[di(2-pyridinthiolato) aluminium]oxy-benzyl}phenyl-N,N'-dioxide group,
with the provision that, when m = 2, R does not mean straight-chain $C_{1-22}$-alkyl.

2. Compounds according to Claim 1, wherein m = 2 and n = 1 and R means phenoxy-monosubstituted straight-chain or branched $C_{1-4}$-alkyl, 2,4-hexadienoyl, optionally with phenyl substituted with 1-5 chlorine atoms, a group having the formula (a) given in Claim 1, or the 2,4,5-trichloro-6-{2,3,5-trichloro-6-[di(2-pyridinthiolato)aluminium]oxybenzyl}phenyl-N,N'-dioxide group.

3. Compounds according to Claim 1, wherein m means the number 1 and n the number 2.

4. (2-phenoxyethoxy)bis(2-pyridinthiolato)aluminium-N,N'-dioxide.

5. Bis(ethylphosphonato) (2-pyridinthiolato)aluminium N-oxide.

6. (2-biphenylyloxy)bis(2-pyridinthiolato)aluminium-N,N'-dioxide.

7. [Methylene-bis(3,4,6-trichloro-o-phenylenoxy]bis[(di-2-pyridinthiolato)]aluminium-N,N',N'',N'''-tetroxide.

8. A compound according to Claim 1, selected from :

(2,4,6-tichlorophenoxy)bis(2-pyridinthiolate)aluminium-N,N'-dioxide,
[methylene-bis(3,4,6-trichloro-o-phenylenoxy](2-pyridinthiolato)aluminium-N-oxide,
bis(2-phenoxyethoxy)(2-pyridinthiolato)aluminium-N-oxide,
diphenoxy(2-pyridinthiolato)aluminium-N-oxide,
bis(2-biphenylyloxy)(2-pyridinthiolato)aluminium-N-oxide,
(sorbato)bis(2-pyridinthiolato)aluminium-N,N'-dioxide,
phenoxy-bis(2-pyridinthiolato)aluminium-N,N'-dioxide and
pentachlorophenoxy-bis(2-pyridinthiolato)aluminium-N,N'-dioxide.

9. A compound according to Claim 1, selected from :

(n-butylphosphonato)bis(2-pyridinthiolato)aluminium-N,N'-dioxide,
(ethylphosphonato)bis(2-pyridinthiolato)aluminium N-oxide and,
bis(n-butylphosphonato)(2-pyridinthiolato)aluminium-N-oxide.

10. Compounds according to any one of Claims 1 to 9 as antimicrobial agents.

11. A cosmetic agent, characterized in that it contains an antimicrobially effective quantity of at least one compound in accordance with Claims 1 to 9.

12. A cosmetic agent according to Claim 11, characterized in that it contains an antimicrobially effective quantity of (2-phenoxyethoxy)bis(2-pyridinthiolato)aluminium-N,N'-dioxide.

13. A cosmetic agent according to Claim 11, characterized in that it contains an antimicrobially effective quantity of bis(ethylphosphonato)(2-pyridinthiolato)aluminium-N-oxide.

14. A cosmetic agent according to Claim 11, characterized in that it contains an antimicrobially effective quantity of (2-biphenyllyloxy)-bis-(2-pyridinthiolato)aluminium-N,N'-dioxide.

15. A cosmetic agent according to Claim 11, characterized in that it contains and antimicrobially effective quantity of [methylene-bis(3,4,6-trichloro-o-phenylenoxy)]-bis-[(di-2-pyridinthiolato)]aluminium-N,N',N'',N'''-tetroxide.

16. A method of producing the compounds according to Claim 1, characterized in that

a) a compound having the formula

$$(R^2)_3Al \qquad (II)$$

wherein $R^2$ means a saturated straight-chain or branched $C_{2-6}$-hydrocarbon remnant, is reacted with a compound having the formula

$$ROH \qquad (III)$$

wherein R has the stated meaning, and with pyridine-N-oxide-2-thiol, or

b) a compound having the formula

$$(R^2)_mAlCl_n \qquad (IV)$$

wherein $R^2$, m and n have the stated meanings, is reacted with a compound having the formula

$$RO^{\ominus}Y^{\oplus} \qquad (V)$$

wherein $Y^{\oplus}$ means an alkaline metal ion or an ammonium ion, and R has the above-stated meaning, and with pyridine-N-oxide-2-thiol.

17. A method of combating or preventing scalp scurf, characterized in that the scalp is treated with an effective quantity of one of the cosmetic agents named in Claims 11 to 15.

18. The use of the compounds of Claims 1 to 9 as agents for care of the hair and feet.

**Revendications**

1. Composés de formule générale

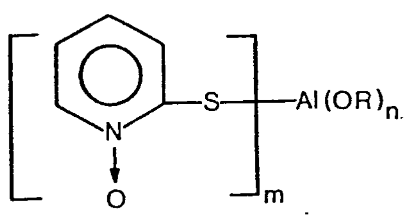

(I)

dans laquelle

m est mis pour le nombre 1 ou 2,

n pour le nombre 1 ou 2,

la somme m + n étant égale à 3, et

R est un alcoyle en $C_{1-22}$ à chaîne droite ; un alcoyle en $C_{1-4}$ à chaîne droite ou ramifié, monosubstitué avec un phénoxy ; un 2,4-hexadiénoyle ; un phényle non substitué ou substitué avec 1 à 5 atomes de chlore ou un radical phényle ; ou un groupe de formule

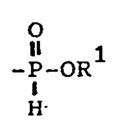

(a)

dans laquelle $R^1$ est un alcoyle en $C_{1-6}$, ou bien, dans le cas où m est mis pour le nombre 1 et n pour le nombre 2, les deux radicaux R forment ensemble le groupe 2,2'-méthylène-bis-(3,4,6-trichloro-o-phénylène) ou encore, dans le cas où m est mis pour le nombre 2 et n pour le nombre 1, R représente en outre le groupe N,N'-bioxyde de 2,4,5-trichloro-6-{2,3,5-trichloro-6-[di-(2-pyridinothiolato)-aluminium]-oxy-benzyl}phényle,

avec cette condition que, quand m = 2, R ne représente pas un alcoyle en $C_{1-22}$ à chaîne droite.

2. Composés selon la revendication 1, caractérisés en ce que m = 2 et n = 1 et en ce que R représente un alcoyle en $C_{1-4}$ à chaîne droite ou ramifié, monosubstitué avec un phénoxy, le groupe 2,4-hexadiénoyle, un phényle éventuellement substitué avec 1 à 5 atomes de chlore, un groupe répondant à la formule (a) donnée dans la revendication 1 ou le groupe N,N'-bioxyde de 2,4,5-trichloro-6-{2,3,5-trichloro-6-[di-(2-pyridinothiolato)-aluminium]-oxy-benzyl}phényle.

3. Composés selon la revendication 1, caractérisés en ce que m est mis pour le nombre 1 et n pour le nombre 2.

4. N,N'-bioxyde de (2-phénoxyéthoxy)-bis-(2-pyridinothiolato)-aluminium.

5. N-oxyde de bis-(éthylphosphonato)-(2-pyridinothiolato)-aluminium.

6. N,N'-bioxyde de (2-biphénylyloxy)-bis-(2-pyridinothiolato)-aluminium.

7. N,N',N'',N'''-tétraoxyde de [méthylène-bis-(3,4,6-trichloro-o-phénylènoxy])-bis-[(di-2-pyridinothiolato)]-aluminium.

8. Composé selon la revendication 1, choisi parmi :

le N,N'-bioxyde de (2,4,6-trichlorophénoxy)-bis-(2-pyridino-thiolato)-aluminium,
le N-oxyde de [méthylène-bis-(3,4,6-trichloro-o-phénylènoxy)]-(2-pyridinothiolato)-aluminium,
le N-oxyde de bis-(2-phénoxyéthoxy)-(2-pyridinothiolato)-aluminium,
le N-oxyde de diphénoxy-(2-pyridinothiolato)-aluminium,
le N-oxyde de bis-(2-biphénylyloxy)-(2-pyridinothiolato)-aluminium,
le N,N'-bioxyde de (sorbato)-bis-(2-pyridinothiolato)-aluminium.
le N,N'-bioxyde de phénoxy-bis-(2-pyridinothiolato)-aluminium et
le N,N'-bioxyde de pentachlorophénoxy-bis-(2-pyrimidothiolato)-aluminium.

9. Composé selon la revendication 1, choisi parmi :

le N,N'-bioxyde de (n-butylphosphonato)-bis-(2-pyridinothiolato)-aluminium,
le N-oxyde d'(éthylphosphonato)-bis-(2-pyridinothiolato)-aluminium et
le N-oxyde de bis-(n-butylphosphonato)-(2-pyridinothiolato)-aluminium.

10. Composés selon l'une quelconque des revendications 1 à 9, en tant qu'agent antimicrobien.

11. Produit cosmétique, caractérisé en ce qu'il contient une quantité suffisante pour avoir une action antimicrobienne d'au moins l'un des composés selon l'une quelconque des revendications 1 à 9.

12. Produit cosmétique selon la revendication 11, caractérisé en ce qu'il contient une quantité suffisante pour avoir une action antimicrobienne de N,N'-bioxyde de (2-phénoxyéthoxy)-bis-(2-pyridinothiolato)-aluminium.

13. Produit cosmétique selon la revendication 11, caractérisé en ce qu'il contient une quantité suffisante pour avoir une action antimicrobienne de N-oxyde de bis-(éthylphosphonato)-(2-pyridinothiolato)-aluminium.

14. Produit cosmétique selon la revendication 11, caractérisé en ce qu'il contient une quantité suffisante pour avoir une action antimicrobienne de N,N'-bioxyde de (2-biphénylyloxy)-bis-(2-pyridinothiolato)-aluminium.

15. Produit cosmétique selon la revendication 11, caractérisé en ce qu'il contient une quantié suffisante pour avoir une action antimicrobienne de N,N',N'',N'''-tétraoxyde de [méthylène-bis-(3,4,6-trichloro-o-phénylènoxy)]-bis[(di-2-pyridinothiolato)]-aluminium.

16. Procédé pour la préparation des composés selon la revendication 1, caractérisé en ce que :

a) on fait réagir un composé de formule

$$(R^2)_3Al \qquad (II)$$

dans laquelle $R^2$ représente un radical hydrocarboné en $C_{2-6}$ saturé, à chaîne droite ou ramifié, avec un composé de formule

$$ROH \qquad (III)$$

dans laquelle R a la signification déjà donnée, et avec le pyridino-N-oxydo-2-thiol, ou

b) on fait réagir un composé de formule

$$(R^2)_mAlCl_n \qquad (IV)$$

dans laquelle $R^2$, m et n ont les significations déjà données, avec un composé de formule

$$RO^{\ominus}Y^{\oplus} \qquad (V)$$

dans laquelle $Y^{\oplus}$ représente un ion métal alcalin ou un ion ammonium et R a la signification déjà donnée, et avec le pyridino-N-oxydo-2-thiol.

17. Procédé pour combattre ou prévenir la formation de pellicules du cuir chevelu, caractérisé en ce qu'on traite le cuir chevelu avec une quantité efficace de l'un des produits cosmétiques selon les revendications 11 à 15.

18. Utilisation des composés selon l'une quelconque des revendications 1 à 9 comme produit de soins des cheveux ou des pieds.